(19) Europäisches Patentamt
European Patent Office
Office européen des brevets

(11) Veröffentlichungsnummer: **0 184 738**
**A2**

(12) # EUROPÄISCHE PATENTANMELDUNG

(21) Anmeldenummer: **85115212.4**

(22) Anmeldetag: **30.11.85**

(51) Int. Cl.⁴: **C 07 D 487/04**, C 07 D 471/04, C 07 D 473/28, C 07 D 473/00, C 07 D 473/04, C 07 D 473/30, A 61 K 31/495, A 61 K 31/435, A 61 K 31/52 // (C07D487/04, 237:00, 235:00), (C07D471/04, 235:00, 221:00)

(30) Priorität: **12.12.84 DE 3445299**

(43) Veröffentlichungstag der Anmeldung: **18.06.86 Patentblatt 86/25**

(84) Benannte Vertragsstaaten: **AT BE CH DE FR GB IT LI LU NL SE**

(71) Anmelder: **Dr. Karl Thomae GmbH, Postfach 1755, D-7950 Biberach (Riss) (DE)**

(72) Erfinder: **Heider, Joachim, Dr. Dipl.-Chem., Am Hang 3, D-7951 Warthausen 1 (DE)**
Erfinder: **Hauel, Norbert, Dr. Dipl.-Chem., Händelstrasse 12, D-7950 Biberach 1 (DE)**
Erfinder: **Austel, Volkhard, Dr. Dipl.-Chem., Kapellenweg 7, D-7950 Biberach 1 (DE)**
Erfinder: **van Meel, Jacques, Dr., Amriswilstrasse 7, D-7950 Biberach 1 (DE)**
Erfinder: **Diederen, Willi, Dr., Haldenstrasse 1a, D-7950 Biberach 1 (DE)**

(54) **Neue Imidazoderivate, diese Verbindungen enthaltende Arzneimittel und Verfahren zu ihrer Herstellung.**

(57) Die Erfindung betrifft neue Imidazoderivate der allgemeinen Formel

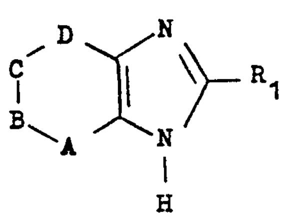 , (I)

in der
einer der Reste B oder C oder auch, wenn mindestens einer der übrigen Reste A, B, C oder D ein Stickstoffatom, eine Imino-, Hydroxymethin-, Alkoxymethin-, Halogenmethin- oder Carbonylgruppe darstellt, A ein Stickstoffatom oder eine Iminogruppe,
ein weiterer der Reste A, B, C oder D ein Stickstoffatom, eine Methin- oder Iminogruppe und
die übrigen der Reste A, B, C oder D, die gleich oder verschieden sein können, Methin-, Hydroxymethin-, Alkoxymethin-, Halogenmethin- oder Carbonylgruppen und
$R_1$ eine gegebenenfalls durch ein Halogenatom, eine Hydroxy-, Alkoxy- oder Alkylmercaptogruppe substituierte Naphthylgruppe bedeuten, deren Tautomere und deren Säureadditionssalze, insbesondere deren physiologisch verträgliche Säureadditionssalze mit anorganischen oder organischen Säuren.

Die neuen Verbindungen weisen wertvolle pharmakologische Eigenschaften auf, insbesondere antithrombotische und cardiovasculäre Eigenschaften wie eine cardiotonische und/oder eine Wirkung auf den Blutdruck, und lassen sich nach an und für sich bekannten Verfahren herstellen.

EP 0 184 738 A2

0184738

DR. KARL THOMAE GMBH
D-7950 Biberach 1

Case 5/915
Dr.Fl./Kp.

# Neue Imidazoderivate, diese Verbindungen enthaltende Arzneimittel und Verfahren zu ihrer Herstellung

In der DE-A1-3.225.386 werden bereits Imidazo[4,5-b]pyridine, die in 2-Stellung durch einen gegebenenfalls in 3-Stellung substituierten Naphth-2-ylrest substituiert sind, beschrieben. Diese besitzen wertvolle pharmakologische Eigenschaften, nämlich eine positiv inotrope Wirkung.

Es wurde nun gefunden, daß die neuen Imidazoderivate der allgemeinen Formel

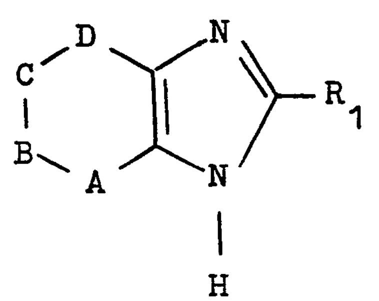

,(I)

deren Tautomere und deren Säureadditionssalze, insbesondere deren physiologisch verträgliche Säureadditionssalze mit anorganischen oder organischen Säuren, welche sich von den bekannten Imidazoderivaten immer durch die Reste A, B, C und/ oder D unterscheiden, überlegene pharmakologische Eigenschaften aufweisen, insbesondere antithrombotische und cardiovasculäre Eigenschaften wie eine cardiotonische und/ oder eine Wirkung auf den Blutdruck.

In der obigen allgemeinen Formel I bedeutet

einer der Reste B oder C oder auch, wenn mindestens einer der übrigen Reste A, B, C oder D ein Stickstoffatom, eine Imino-, Hydroxymethin-, Alkoxymethin-, Halogenmethin- oder Carbonylgruppe darstellt, A ein Stickstoffatom oder eine Iminogruppe,
ein weiterer der Reste A, B, C oder D ein Stickstoffatom, eine Methin- oder Iminogruppe und
die übrigen der Reste A, B, C oder D, die gleich oder verschieden sein können, Methin-, Hydroxymethin-, Alkoxymethin-, Halogenmethin- oder Carbonylgruppen und

$R_1$ eine gegebenenfalls durch ein Halogenatom, eine Hydroxy-, Alkoxy- oder Alkylmercaptogruppe substituierte Naphthylgruppe, wobei der Alkylteil jeweils 1 bis 3 Kohlenstoffatome enthalten kann.

Gegenstand der vorliegenden Anmeldung sind somit die in 2-Stellung des Imidazolringes entsprechend substituierten Imidazo[4,5-c]pyridazin-6-one, Imidazo[4,5-d]pyridazin-4-one, Imidazo[4,5-c]pyridine, Imidazo[4,5-c]pyridin-4-one, Imidazo[4,5-b]pyridin-5-one, Imidazo[4,5-c]pyridin-7-one, 5-Alkoxy-imidazo[4,5-b]pyridine, Purine, Purin-2-one, 5-Hydroxy-imidazo[4,5-b]pyridine, 2-Alkoxy-purine, 2-Chlor-purine, 2-Brom-purine, Purin-6-one, Purin-2,6-dione, 6-Alkoxy-purine, 6-Chlor-purine, 6-Brom-purine, 5-Chlor-imidazo-[4,5-b]pyridine und 5-Brom-imidazo[4,5-b]pyridine.

Für die bei der Definition der Reste A bis D eingangs erwähnten Halogenatom kommt insbesondere das Chlor- oder Bromatom,

für die eingangs erwähnten Alkoxygruppen insbesondere die Methoxy-, Äthoxy-, n-Propoxy- oder Isopropoxygruppe und

für $R_1$ die Naphth-1-yl-, Naphth-2-yl-, 3-Hydroxy-naphth-2-yl-, 3-Methoxy-naphth-2-yl-, 3-Äthoxy-naphth-2-yl-, 3-n-Propoxy-naphth-2-yl-, 3-Isopropoxy-naphth-2-yl-, 3-Fluor-naphth-2-yl-, 3-Chlor-naphth-2-yl-, 3-Brom-naphth-2-yl-, 3-Jod-naphth-2-yl-, 3-Methylthio-naphth-2-yl-, 3-n-Propylthio-naphth-2-yl-, 6-Hydroxy-naphth-2-yl-, 6-Methoxy-naphth-2-yl-, 7-Chlor-naphth-2-yl-, 6-Brom-naphth-2-yl- oder 6-Methylthio-naphth-2-yl-gruppe in Betracht.

Bevorzugte Verbindungen der obigen allgemeinen Formel I sind jedoch die entsprechend substituierte Imidazo[4,5-c]pyridine, Imidazo[4,5-b]pyridin-5-one, 5-Methoxy-imidazo[4,5-b]-pyridine, Imidazo[4,5-c]pyridin-4-one, Purine, Purin-2-one, 2-Methoxy-purine, Purin-2,6-dione, 5-Chlor-imidazo[4,5-b]-pyridine, 2-Chlor-purine und Imidazo[4,5-d]pyridazin-4-one der obigen allgemeinen Formel I, wobei $R_1$ eine in 3-Stellung gegebenenfalls durch ein Chloratom, eine Hydroxy-, Methoxy-, n-Propoxy- oder Methylthiogruppe substituierte Naphth-2-yl-gruppe darstellt, deren Tautomere und deren Säureadditionssalze, insbesondere deren physiologisch verträgliche anorganische oder organische Säureadditionssalze.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

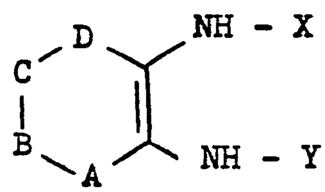

,(II)

in der
A bis D wie eingangs definiert sind,
einer der Reste X oder Y ein Wasserstoffatom und der andere der beiden Reste X und Y oder beide Reste X und Y eine Gruppe der Formel

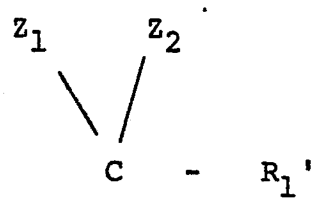

darstellen, in der

$R_1$' die für $R_1$ eingangs erwähnten Bedeutungen besitzt oder eine durch eine Hydroxygruppe substituierte Naphthylgruppe, in der die Hydroxygruppe durch einen Schutzrest geschützt ist,
$Z_1$ und $Z_2$, die gleich oder verschieden sein können, gegebenenfalls substituierte Aminogruppe oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin, Pyridin oder in einem Überschuß des zur Herstellung der Verbindung der allgemeinen Formel II verwendeten Acylierungsmittel, z.B. in dem entsprechenden Nitril, Anhydrid, Säurehalogenid, Ester, Amid oder Methojodid, beispielsweise bei Temperturen zwischen 0 und 250°C, vorzugszweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Natriumhydroxid, Kaliumhydroxid, Kaliumäthylat oder Kalium-tert.butylat durchgeführt. Die Cyclisierung kann jedoch auch ohne Lösungsmittel und/oder Kondensationsmittel durchgeführt werden.

b) Zur Herstellung von Verbindungen der allgemeinen Formel I, in der mindestens einer der Reste A, B, C oder D eine Carbonylgruppe darstellt:

Abspaltung eines Restes von einer Verbindung der allgemeinen Formel

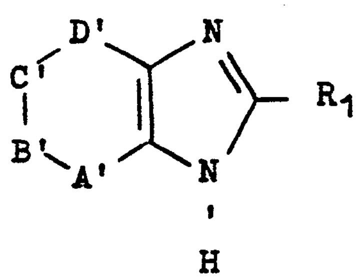

,(III)

in der

$R_1$ wie eingangs definiert ist und
mindestens einer der Reste A', B', C' oder D' eine Halogenmethingruppe oder eine durch eine Schutzgruppe geschützte Hydroxymethingruppe wie die Methoxymethin-, Äthoxymethin- oder Benzoyloxymethingruppe und
die übrigen der Reste A', B', C' oder D' die für A, B, C oder D eingangs erwähnten Bedeutungen besitzen.

Die Abspaltung eines Schutzrestes erfolgt je nach dem verwendeten Rest entweder mittels Hydrolyse oder mittels Hydrogenolyse gegebenenfalls in einem geeigneten Lösungsmittel bei Temperaturen zwischen -78 und 250°C.

Beispielsweise erfolgt die Ätherspaltung in Gegenwart einer Säure wie Chlorwasserstoff, Bromwasserstoff, Schwefelsäure, Bortribromid, Aluminiumtrichlorid oder Pyridinhydrochlorid zweckmäßigerweise in einem geeigneten Lösungsmittel wie Methylenchlorid, Eisessig oder Wasser oder in deren Gemischen bei Temperaturen zwischen -78 und 250°C. Hierbei wird die Ätherspaltung in Gegenwart einer Protonensäure wie Salzsäure, wobei diese gleichzeitig auch als Lösungsmittel dienen kann, zweckmäßigerweise bei Temperaturen zwischen 50 und 175°C, vorzugsweise bei Temperaturen zwischen 100 und 150°C, oder mit einer Lewis-Säure vorzugsweise in einem Lösungsmittel wie Methylenchlorid bei Temperaturen zwischen -78 und 20°C, durchgeführt.

Beispielsweise erfolgt der Halogenaustausch in Gegenwart einer Base wie Natriumhydroxid, Kaliumhydroxid, Ammoniak oder Natriumkarbonat in einem wässrigen Lösungsmittel, z.B. in Wasser, Wasser/Tetrahydrofuran oder Wasser/Dioxan bei Temperaturen zwischen 50 oder 100°C, vorzugsweise jedoch bei der Siedetemperatur des verwendeten Lösungsmittels.

Beispielsweise erfolgt die Esterspaltung in Gegenwart einer Base wie wässrigem Ammoniak, Natriumkarbonat, Kaliumcarbonat, Natrium- oder Kaliumhydroxid in einem Lösungsmittel wie Wasser, Wasser/Methanol, Wasser/Äthanol, Wasser/Tetrahydrofuran oder Wasser/Dioxan bei Temperaturen zwischen 50 und 100°C, vorzugsweise bei der Siedetemperatur des verwendeten Lösungsmittels.

Beispielsweise erfolgt die hydrogenolytische Abspaltung einer verwendeten Schutzgruppe wie der Benzylgruppe mit Wasserstoff in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid, vorzugsweise z.B. bei Raumtemperatur, und einem Wasserstoffdruck von 1 bis 5 bar.

Die erfindungsgemäß erhaltenen Verbindungen der allgemeinen Formel I können gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis IV sind teilweise literaturbekannt bzw. erhält man nach literaturbekannten Verfahren.

So erhält man beispielsweise die als Ausgangsstoffe verwendeten Verbindungen der allgemeinen Formel II durch Acylierung einer entsprechenden o-Diaminoverbindung und die Verbindungen der allgemeinen Formel IV durch Acylierung einer entsprechenden o-Diaminoverbindung und anschließende Cyclisierung mit einem entsprechenden Naphthylsäurederivat (siehe EP-A-0.022.495).

Wie bereits eingangs erwähnt weisen die neuen Verbindungen der allgemeinen Formel I, deren 1H Tautomere und deren physiologisch verträgliche Säureadditionssalze überlegene pharmakologische Eigenschaften auf, insbesondere antithrombotische und cardiovasculäre Eigenschaften wie eine cardiotonische und/oder eine Wirkung auf den Blutdruck.

Beispielsweise wurden die Verbindungen

A = 2-(Naphth-2-yl)-5H-imidazo[4,5-d]pyridazin-4-on,

B = 2-(3-Chlor-naphth-2-yl)-1H-imidazo[4,5-b]pyridin-5-on,

C = 2-Methoxy-8-(naphth-2-yl)-purin und

D = 2-Chlor-8-(3-n-propoxy-naphth-2-yl)-purin

auf ihre biologischen Eigenschaften wie folgt untersucht:

Bestimmung der positiv inotropen Wirkung an despinalisierten Meerschweinchen

Die Untersuchungen wurden an despinalisierten Meerschweinchen durchgeführt. Die Tiere wurden mit 50 % $O_2$ + 50 % $N_2$ beatmet. Der arterielle Blutdruck wurde in der rechten Arteria Carotis mit einem Bell and Howell Druckwandler (4-327-I) gemessen. Für die Erfassung der positiv inotropen Wirkung wurde mit einem Kathetertipmanometer (Millar PR-249) der Druck in dem linken Ventrikel (LV) gemessen. Mittels eines Differenzierers wurde LV-dp/dtmax gewonnen. Die zu untersuchenden Substanzen wurden in eine Vena jugularis injiziert. Als Lösungsdmittel diente 0,9 % NaCl+Polydiol 200 (1:1).
Jede Substanz wurde an 3 Meerschweinchen überprüft. Die Dosen waren 0,1 - 3 mg/kg i.v.. Die nachfolgende Tabelle enthält die Mittelwerte bei 1 mg/kg i.v..

| Substanz | Dosis mg/kg i.v. | Zunahme in LV-dp/dtmax |
|---|---|---|
| A | 1 | 28 % |
| B | 1 | 30 % |
| C | 1 | 33 % |
| D | 1 | 27 % |

Die neuen Verbindungen sind gut verträglich, so konnte bei der Untersuchung der Substanzen A bis D bis zu einer Dosis von 3 mg/kg i.v. keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern und zum Teil durch Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,1 - 15 mg/kg Körpergewicht, vorzugsweise jedoch 3 - 10 mg/kg Körpergewicht.

Die nachfolgenden Beispiele sollen die Erfindung näher erläutern:

## Beispiel 1

### 2-(Naphth-2-yl)-5H-imidazo[4,5-d]pyridazin-4-on

2,5 g 4,5-Diamino-2H-pyridazin-3-on werden mit 3,4 g Naphthalin-2-carbonsäure verrieben und in 100 g Polyphosphorsäure eingerührt. Man erhitzt 3 Stunden auf 120-130°C, gießt dann auf ca. 500 ml Eiswasser und stellt mit wässrigem Ammoniak schwach alkalisch. Der ausgefallene Niederschlag wird abgesaugt, in Äthanol gelöst und über Aktivkohle filtriert. Nach dem Eindampfen des Äthanols wird der Rückstand 2 x aus wenig Dimethylformamid umkristallisiert.
Ausbeute: 1,2 g (23 % der Theorie),
Schmelzpunkt: > 300°C

| | | | | | | |
|---|---|---|---|---|---|---|
| Ber.: | C | 68,69 | H | 3,84 | N | 21,36 |
| Gef.: | | 68,60 | | 3,94 | | 21,69 |

## Beispiel 2

### 2-(Naphth-2-yl)-1H-imidazo[4,5-c]pyridin-4-on

a) 3-(3-Methoxy-naphth-2-carbamido)-4-amino-pyridin-2-on

0,64 g Naphthalin-2-carbonsäure werden in 10 ml Dimethylformamid gelöst und 0,65 g N,N'-Carbonyldiimidazol zugegeben. Man rührt 45 Minuten bei Raumtemperatur nach und gibt 0,6 g 3,4-Diamino-pyridin-2-on hinzu. Die Mischung wird 2 Stunden bei Raumtemperatur gerührt und anschließend vom Lösungsmittel abdestilliert. Der Rückstand wird mit Wasser verrieben, abgesaugt und mit Wasser gewaschen. Das erhaltene Produkt wird an der Luft getrocknet.
Ausbeute: 0,89 g (86 % der Theorie),
Schmelzpunkt: 255-258°C

b) 3-(Naphth-2-yl)-1H-imidazo[4,5-c]pyridin-4-on

Hergestellt aus 0,85 g 3-(3-Methoxy-naphth-2-carbamido)-4-amino-pyridin-2-on und Polyphosphorsäure analog Beispiel 1.
Ausbeute: 0,2 g (18 % der Theorie)
Schmelzpunkt: > 310°C

| | | | | | | |
|---|---|---|---|---|---|---|
| Ber.: | C | 73,55 | H | 4,24 | N | 16,08 |
| Gef.: | | 73,86 | | 4,41 | | 15,76 |

Beispiel 3

5-Methoxy-2-(3-methoxy-naphth-2-yl)-1H-imidazo[4,5-b]-pyridin

2,1 g 2,3-Diamino-6-methoxy-pyridin werden mit 2,0 g 3-Methoxy-naphthalin-2-carbonsäure verrieben und in 50 ml Phosphoroxychlorid eingerührt. Man erhitzt das Gemisch 2 Stunden zum Rückfluß und zersetzt anschließend auf ca. 300 g Eiswasser. Der Niederschlag wird abgesaugt, in ca. 100 ml 5%iger Natriumhydrogencarbonatlösung 10 Minuten lang gerührt und nach dem Absaugen bei 70°C im Umlufttrockenschrank getrocknet. Die Reinigung erfolgt durch Säulenchromatographie (Elutionsmittel: Methylenchlorid/ 0-2 % Äthanol).
Ausbeute: 1,65 g (54 % der Theorie),
Schmelzpunkt: 204-205°C

| | | | | | | |
|---|---|---|---|---|---|---|
| Ber.: | C | 70,80 | H | 4,95 | N | 13,76 |
| Gef.: | | 71,07 | | 4,98 | | 13,87 |

## Beispiel 4

### 2-(3-Methoxy-naphth-2-yl)-1H-imidazo[4,5-b]pyridin-5-on

0,6 g 5-Methoxy-2-(3-methoxy-naphth-2-yl)-1H-imidazo[4,5-b]-pyridin werden zusammen mit 50 ml konzentrierter Salzsäure 35 Stunden in einem Druckgefäß auf 130°C erhitzt. Nach dem Abkühlen wird eingedampft, der Rückstand in ca. 50 ml 2 N Natronlauge gelöst und über Aktivkohle filtriert. Der beim Ansäuern mit Salzsäure erhaltene Niederschlag wird abgesaugt, mit Wasser gewaschen und im Umlufttrockenschrank bei 70°C getrocknet.
Ausbeute: 0,55 g (94,5 % der Theorie),
Schmelzpunkt: > 300°C

| | | | | | | |
|---|---|---|---|---|---|---|
| Ber.: | C | 70,09 | H | 4,50 | N | 14,42 |
| Gef.: | | 70,25 | | 4,31 | | 14,68 |

## Beispiel 5

### 2-Methoxy-8-(naphth-2-yl)-purin

a) 2-Methoxy-4-amino-5-(naphth-2-carbamido)-pyrimidin

Hergestellt aus 3,4 g Naphthalin-2-carbonsäure und 3,3 g N,N'-Carbonyldiimidazol, gelöst in 75 ml Dimethylformamid, und 2,8 g 2-Methoxy-4,5-diamino-pyrimidin bei 100°C analog Beispiel 2a.
Ausbeute: 2,3 g (39 % der Theorie),
Schmelzpunkt: 195-198°C

b) 2-Methoxy-8-(naphth-2-yl)-purin

2,3 g 2-Methoxy-4-amino-5-(naphth-2-carbamido)-pyrimidin

werden in 50 ml 20%iger Natronlauge und 50 ml Äthanol 6 Stunden am Rückfluß gekocht. Nach Entfernen des Äthanols säuert man mit konzentrierter Salzsäure an und saugt den Niederschlag ab, welcher bei 70°C im Umlufttrockenschrank getrocknet wird.
Ausbeute: 1,2 g (56 % der Theorie),
Schmelzpunkt: 282-284°C

| | | | | | | |
|---|---|---|---|---|---|---|
| Ber.: | C | 69,55 | H | 4,38 | N | 20,27 |
| Gef.: | | 69,87 | | 4,41 | | 19,86 |

Beispiel 6

8-(Naphth-2-yl)-purin-2-on

Hergestellt aus 1,0 g 2-Methoxy-8-(naphth-2-yl)-purin analog Beispiel 4.
Ausbeute: 0,35 g (37 % der Theorie)
Schmelzpunkt: > 300°C

| | | | | | | |
|---|---|---|---|---|---|---|
| Ber.: | C | 68,69 | H | 3,84 | N | 21,36 |
| Gef.: | | 68,88 | | 4,05 | | 21,47 |

Beispiel 7

2-(3-Methoxy-naphth-2-yl)-5H-imidazo[4,5-d]pyridazin-4-on

Hergestellt aus 1,3 g 3-Methoxy-naphthalin-2-carbonsäure und 0,8 g 4,5-Diamino-2H-pyridazin-3-on in 500 g Polyphosphorsäure analog Beispiel 1.
Ausbeute: 0,4 g (21 % der Theorie),
Schmelzpunkt: > 300°C

| | | | | | | |
|---|---|---|---|---|---|---|
| Ber.: | C | 65,75 | H | 4,14 | N | 19,17 |
| Gef.: | | 65,60 | | 4,21 | | 18,88 |

Beispiel 8

8-(3-Methoxy-naphth-2-yl)-purin

Hergestellt aus 2,0 g 3-Methoxy-naphthalin-2-carbonsäure und 1,5 g 4,5-Diamino-pyrimidin-hydrochlorid in 30 g Polyphosphorsäure analog Beispiel 1.
Ausbeute: 0,6 g (22 % der Theorie),
Schmelzpunkt: 209-210°C

| | | | | | | |
|---|---|---|---|---|---|---|
| Ber.: | C | 69,55 | H | 4,38 | N | 20,28 |
| Gef.: | | 69,80 | | 4,54 | | 20,05 |

Beispiel 9

2-(3-Methoxy-naphth-2-yl)-1H-imidazo[4,5-c]pyridin

Hergestellt aus 2,0 g 3-Methoxy-naphthalin-2-carbonsäure und 1,1 g 3,4-Diamino-pyridin in 30 g Polyphosphorsäure analog Beispiel 1.
Ausbeute: 1,1 g (40 % der Theorie),
Schmelzpunkt: 203-204°C

| | | | | | | |
|---|---|---|---|---|---|---|
| Ber.: | C | 74,17 | H | 4,76 | N | 15,26 |
| Gef.: | | 74,08 | | 4,89 | | 14,89 |

Beispiel 10

2-(3-Methoxy-naphth-2-yl)-1H-imidazo[4,5-c]pyridin-4-on

a) 3-(3-Methoxy-naphth-2-carbamido)-pyridin-2-on

Hergestellt aus 2,0 g 3-Methoxy-naphthalin-2-carbonsäure mit 1,8 g N,N'-Carbonyldiimidazol in 20 ml Dimethylformamid und

1,6 g 3,4-Diamino-pyridin-2-on analog Beispiel 2a.
Ausbeute: 1,6 g (52 % der Theorie),
Schmelzpunkt: > 250°C

| | | | | | | |
|---|---|---|---|---|---|---|
| Ber.: | C | 66,01 | H | 4,89 | N | 13,59 |
| Gef.: | | 65,94 | | 4,86 | | 13,48 |

b) <u>2-(3-Methoxy-naphth-2-yl)-1H-imidazo[4,5-c]pyridin-4-on</u>

Hergestellt aus 1,4 g 3-(3-Methoxy-naphth-2-carbamido)-pyridin-2-on und 5 g Polyphosphorsäure analog Beispiel 1.
Ausbeute: 0,09 g (6 % der Theorie),
Schmelzpunkt: 226-229°C

| | | | | | | |
|---|---|---|---|---|---|---|
| Ber.: | C | 62,29 | H | 4,31 | N | 12,82 |
| Gef.: | | 61,95 | | 4,47 | | 12,85 |

## Beispiel 11

### 5-Methoxy-2-(naphth-2-yl)-1H-imidazo[4,5-b]pyridin

Hergestellt aus 3,2 g 2,3-Diamino-6-methoxy-pyridin und 2,6 g Naphthalin-2-carbonsäure in 100 ml Phosphoroxychlorid analog Beispiel 3.
Ausbeute: 2,15 g (52 % der Theorie),
Schmelzpunkt: 117-119°C

| | | | | | | |
|---|---|---|---|---|---|---|
| Ber.: | C | 74,16 | H | 4,76 | N | 15,26 |
| Gef.: | | 74,37 | | 4,71 | | 15,10 |

## Beispiel 12

### 2-(Naphth-2-yl)-1H-imidazo[4,5-b]pyridin-5-on

Hergestellt aus 1,0 g 5-Methoxy-2-(naphth-2-yl)-1H-imidazo[4,5-b]pyridin und konzentrierter Salzsäure analog Beispiel 4.
Ausbeute: 0,48 g (50 % der Theorie),
Schmelzpunkt: > 300°C

| | | | | | | |
|---|---|---|---|---|---|---|
| Ber.: | C | 73,55 | H | 4,24 | N | 16,08 |
| Gef.: | | 73,87 | | 4,35 | | 16,29 |

Beispiel 13

5-Methoxy-2-(3-methylmercapto-naphth-2-yl)-1H-imidazo-[4,5-b]pyridin

Hergestellt aus 2,1 g 2,3-Diamino-6-methoxy-pyridin und 2,2 g 3-Methylmercapto-naphthalin-2-carbonsäure in Phosphoroxychlorid analog Beispiel 3.
Ausbeute: 1,95 g (61 % der Theorie).
Schmelzpunkt: 236-238°C

| | | | | | | | | |
|---|---|---|---|---|---|---|---|---|
| Ber.: | C | 67,26 | H | 4,70 | N | 13,07 | S | 9,97 |
| Gef.: | | 66,98 | | 4,86 | | 12,99 | | 10,19 |

Beispiel 14

2-(3-Methylmercapto-naphth-2-yl)-1H-imidazo[4,5-b]pyridin-5-on

Hergestellt aus 1,5 g 5-Methoxy-2-(3-methylmercapto-naphth-2-yl)-1H-imidazo[4,5-b]pyridin und 50 ml konzentrierter Salzsäure analog Beispiel 4.
Ausbeute: 0,45 g (32 % der Theorie),
Schmelzpunkt: 192-194°C

| | | | | | | | | |
|---|---|---|---|---|---|---|---|---|
| Ber.: | C | 66,43 | H | 4,26 | N | 13,67 | S | 10,43 |
| Gef.: | | 66,37 | | 4,38 | | 13,45 | | 10,69 |

Beispiel 15

5-Methoxy-2-(3-chlor-naphth-2-yl)-1H-imidazo[4,5-b]pyridin

Hergestellt aus 2,1 g 3,4-Diamino-6-methoxy-pyridin und 2,1 g 3-Chlor-naphthalin-2-carbonsäure in 50 ml Phosphoroxychlorid analog Beispiel 3.
Ausbeute: 1,5 g (50 % der Theorie),
Schmelzpunkt: ab 94°C amorph

| | | | | | | | | |
|---|---|---|---|---|---|---|---|---|
| Ber.: | C | 65,91 | H | 3,91 | N | 13,56 | Cl | 11,44 |
| Gef.: | | 66,03 | | 4,07 | | 13,40 | | 11,39 |

Beispiel 16

2-(3-Chlor-naphth-2-yl)-1H-imidazo[4,5-b]pyridin-5-on

Hergestellt aus 0,75 g 5-Methoxy-2-(3-chlor-naphth-2-yl)-1H-imidazo[4,5-b]-pyridin und 50 ml konzentrierter Salzsäure analog Beispiel 4.
Ausbeute: 0,49 g (68 % der Theorie),
Schmelzpunkt: > 350°C

| | | | | | | | | |
|---|---|---|---|---|---|---|---|---|
| Ber.: | C | 64,98 | H | 3,41 | N | 14,21 | Cl | 11,99 |
| Gef.: | | 64,60 | | 3,65 | | 13,94 | | 11,80 |

Beispiel 17

5-Methoxy-2-(3-propoxy-naphth-2-yl)-1H-imidazo[4,5-b]pyridin

Hergestellt aus 3,2 g 2,3-Diamino-6-methoxy-pyridin und 3-Propoxy-naphthalin-2-carbonsäure in 75 ml Phosphoroxychlorid analog Beispiel 3.

Ausbeute: 1,7 g (34 % der Theorie).
Schmelzpunkt: 178-181°C

| | | | | | | |
|---|---|---|---|---|---|---|
| Ber.: | C | 72,05 | H | 5,74 | N | 12,60 |
| Gef.: | | 72,15 | | 5,76 | | 12,92 |

Beispiel 18

2-(3-Hydroxy-naphth-2-yl)-1H-imidazo[4,5-b]pyridin-5-on

Hergestellt aus 1,5 g 5-Methoxy-2-(3-propoxy-naphth-2-yl)-1H-imidazo[4,5-b]-pyridin und 75 ml konzentrierter Salzsäure analog Beispiel 4.
Ausbeute: 0,4 g (32 % der Theorie),
Schmelzpunkt: > 300°C

| | | | | | | |
|---|---|---|---|---|---|---|
| Ber.: | C | 69,30 | H | 4,00 | N | 15,15 |
| Gef.: | | 68,96 | | 4,35 | | 13,44 |

Beispiel 19

2-Methoxy-8-(3-methoxy-naphth-2-yl)-purin-monohydrat

a) 2-Methoxy-4-amino-5-(3-methoxy-naphthalin-2-carbamido)-pyrimidin

Hergestellt aus 4,0 g 3-Methoxy-naphthalin-2-carbonsäure mit 3,6 g N,N'-Carbonyldiimidazol in 50 ml Dimethylformamid und 2,8 g 2-Methoxy-4,5-diamino-pyrimidin bei 100°C analog Beispiel 2a.
Ausbeute: 4,8 g (74 % der Theorie),
Schmelzpunkt: 228-230°C

b) 2-Methoxy-8-(3-methoxy-naphth-2-yl)-purin-dihydrat

Hergestellt aus 4,7 g 2-Methoxy-4-amino-5-(3-methoxy-naphth-2-carbamido)-pyrimidin in 50 ml 20%iger Natronlauge und 50 ml Äthanol analog Beispiel 5b.
Ausbeute: 1,1 g (25 % der Theorie),
Schmelzpunkt: 181-183°C

| | | | | | | |
|---|---|---|---|---|---|---|
| Ber.: | C | 59,63 | H | 5,29 | N | 16,36 |
| Gef.: | | 59,51 | | 5,44 | | 15,94 |

Beispiel 20

8-(3-Methoxy-naphth-2-yl)-purin-2-on-monohydrat

Hergestellt aus 0,85 g 2-Methoxy-8-(3-methoxy-naphth-2-yl)-purin-dihydrat und 17 ml konzentrierter Salzsäure analog Beispiel 4.
Ausbeute: 0,27 g (33 % der Theorie),
Schmelzpunkt: > 300°C

| | | | | | | |
|---|---|---|---|---|---|---|
| Ber.: | C | 61,92 | H | 4,55 | N | 18,05 |
| Gef.: | | 61,80 | | 4,73 | | 17,96 |

Beispiel 21

8-(3-Methoxy-naphth-2-yl)-purin-2,6-dion

a) 2,6-Dihydroxy-4-amino-5-(3-methoxy-naphth-2-carbamido)-pyrimidin

Hergestellt aus 4,0 g 3-Methoxy-naphthalin-2-carbonsäure mit 3,6 g N,N'-Carbonyldiimidazol in 50 ml Dimethylformamid und 2,8 g 2,6-Dihydroxy-4,5-diamino-pyrimidin analog Beispiel 2a.
Ausbeute: 2,3 g (35 % der Theorie),
Schmelzpunkt: > 300°C

b) 8-(3-Methoxy-naphth-2-yl)-purin-2,6-dion

Hergestellt aus 1,3 g 2,6-Dihydroxy-4-amino-5-(3-methoxy-naphthalin-2-carbamido)- pyrimidin analog Beispiel 5b.
Ausbeute: 0,62 g (51 % der Theorie)
Schmelzpunkt: > 300°C

| | | | | | | |
|---|---|---|---|---|---|---|
| Ber.: | C | 62,32 | H | 3,92 | N | 18,17 |
| Gef.: | | 61,90 | | 4,08 | | 18,49 |

Beispiel 22

8-(Naphth-2-yl)-purin-2,6-dion

a) 4-Amino-5-(naphth-2-carbamido)-pyrimidinyl-2,6-dion

Hergestellt aus 5,2 g Naphthalin-2-carbonsäure mit 5,6 g N,N'-Carbonyldiimidazol in 100 ml Dimethylformamid und 4,2 g 2,6-Dihydroxy-4,5-diamino-pyrimidin analog Beispiel 2a.
Ausbeute: 1,7 g (20 % der Theorie),
Schmelzpunkt: > 300°C

b) 8-(Naphth-2-yl)-purin-2,6-dion

Hergestellt aus 2,7 g 2,6-Dihydroxy-4-amino-5-(naphthalin-2-carbamido)pyrimidin analog Beispiel 5b.
Ausbeute: 1,7 g (68 % der Theorie),
Schmelzpunkt: > 300°C

| | | | | | | |
|---|---|---|---|---|---|---|
| Ber.: | C | 64,74 | H | 3,62 | N | 20,13 |
| Gef.: | | 64,51 | | 3,74 | | 20,45 |

Beispiel 23

2-Methoxy-8-(3-chlor-naphth-2-yl)-purin

a) 2-Methoxy-4-amino-5-(3-chlor-naphth-2-carbamido)-pyrimidin

Hergestellt aus 4,2 g 3-Chlor-naphthalin-2-carbonsäure mit 3,6 g N,N'-Carbonyldiimidazol in 50 ml Dimethylformamid und 2,8 g 2-Methoxy-4,5-diamino-pyrimidin bei 100°C analog Beispiel 2a.
Ausbeute: 3,3 g (50 % der Theorie),
Schmelzpunkt: 246-248°C

| | | | | | | | | |
|---|---|---|---|---|---|---|---|---|
| Ber.: | C | 58,45 | H | 3,98 | N | 17,04 | Cl | 10,78 |
| Gef.: | | 58,30 | | 3,71 | | 17,46 | | 10,99 |

b) 2-Methoxy-8-(3-chlor-naphth-2-yl)-purin

Hergestellt aus 3,3 g 2-Methoxy-4-amino-5-(3-chlor-naphth-2-carbamido)-pyrimidin in 50 ml 20%iger Natronlauge und 50 ml Äthanol analog Beispiel 5b.
Ausbeute: 1,2 g (39 % der Theorie),
Schmelzpunkt: 236-238°C

Beispiel 24

8-(3-Chlor-naphth-2-yl)-purin-2-on

Hergestellt aus 1,0 g 2-Methoxy-8-(3-chlor-naphth-2-yl)-purin analog Beispiel 4.
Ausbeute: 0,34 g (36 % der Theorie)
Schmelzpunkt: > 300°C

| | | | | | | | | |
|---|---|---|---|---|---|---|---|---|
| Ber.: | C | 60,71 | H | 3,06 | N | 18,88 | Cl | 11,95 |
| Gef.: | | 60,63 | | 2,91 | | 19,18 | | 11,71 |

Beispiel 25

2-Chlor-8-(3-n-propoxy-naphth-2-yl)-purin

Hergestellt aus 2,3 g 3-n-Propoxy-naphthyl-2-carbonsäure mit 2,2 g 2-Chlor-4,5-diamino-pyrimidin-dihydrochlorid in 50 ml Phosphoroxychlorid analog Beispiel 3.
Ausbeute: 1,4 g (41 % der Theorie),
Schmelzpunkt: 201-203°C

| | | | | | | | | |
|---|---|---|---|---|---|---|---|---|
| Ber.: | C | 63,81 | H | 4,46 | N | 16,54 | Cl | 10,46 |
| Gef.: | | 63,90 | | 4,22 | | 16,43 | | 10,69 |

Beispiel 26

5-Chlor-2-(3-n-propoxy-naphth-2-yl)-1H-imidazo[4,5-b]pyridin

Hergestellt aus 2,7 g 2,3-Diamino-6-chlor-pyridin-hydrochlorid mit 3,5 g 3-n-Propoxy-naphthyl-2-carbonsäure in 75 ml Phosphoroxychlorid analog Beispiel 3.
Ausbeute: 3,0 g (59 % der Theorie),
Schmelzpunkt: 187-189°C

| | | | | | | | | |
|---|---|---|---|---|---|---|---|---|
| Ber.: | C | 67,55 | H | 4,77 | N | 12,44 | Cl | 10,49 |
| Gef.: | | 67,50 | | 4,95 | | 12,20 | | 10,56 |

Beispiel 27

2-Chlor-8-(3-methylmercapto-naphthyl-2)-purin

Hergestellt aus 2,2 g 2-Chlor-4,5-diamino-pyrimidin-dihydrochlorid mit 2,2 g 3-Methylmercapto-naphthyl-2-carbonsäure in 50 ml Phosphoroxychlorid analog Beispiel 3.

Ausbeute: 2,0 g (61 % der Theorie),
Schmelzpunkt: 211-213°C

| | | | | | | | | | | |
|---|---|---|---|---|---|---|---|---|---|---|
| Ber.: | C | 58,80 | H | 3,39 | N | 17,14 | S | 9,81 | Cl | 10,85 |
| Gef.: | | 59,00 | | 3,66 | | 17,30 | S | 9,91 | Cl | 11,00 |

Beispiel 28

8-(3-Methylmercapto-naphth-2-yl)-purin-2-on

1,8 g 2-Chlor-8-(3-methylmercapto-naphth-2-yl)-purin werden mit 180 ml 4 n Kalilauge 36 Stunden am Rückfluß gekocht. Man filtriert die noch warme Lösung über Aktivkohle, säuert mit konz. Salzsäure an und saugt nach dem Abkühlen vom ausgefallenen Niederschlag ab. Die Reinigung erfolgt durch Umkristallisieren aus 500 ml Dimethylformamid.
Ausbeute: 0,65 g (38 % der Theorie),
Schmelzpunkt: > 300°C

| | | | | | | | | |
|---|---|---|---|---|---|---|---|---|
| Ber.: | C | 62,32 | H | 3,92 | N | 18,17 | S | 10,40 |
| Gef.: | | 62,60 | | 4,04 | | 18,24 | S | 10,24 |

Beispiel 29

2-(3-Methylmercapto-naphth-2-yl)-purin-4,6-dion

Hergestellt aus 1,0 g 5-(3-Methylmercapto-naphth-2-carbamido)-6-amino-pyrimidin-2,4-dion und 6 g Kaliumhydroxid in 30 ml Pentanol analog Beispiel 5.
Ausbeute: 0,15 g (16 % der Theorie),
Schmelzpunkt: > 300°C

| | | | | | | | | |
|---|---|---|---|---|---|---|---|---|
| Ber.: | C | 59,24 | H | 3,73 | N | 17,27 | S | 9,89 |
| Gef.: | | 58,77 | | 4,02 | | 17,12 | S | 9,96 |

Beispiel 30

2-(3-n-Propoxy-naphth-2-yl)-purin-6-on-dihydrat

Hergestellt aus 1,6 g 5-(3-n-Propoxy-naphth-2-carbamido)-6-amino-pyrimidin-2-on und 25 ml 2n Natronlauge in 25 ml Äthanol analog Beispiel 5.

Ausbeute: 0,54 g (36 % der Theorie),

Schmelzpunkt: 272-274°C

| | | | | | | |
|---|---|---|---|---|---|---|
| Ber.: | C | 60,66 | H | 5,65 | N | 15,72 |
| Gef.: | | 60,37 | | 5,12 | | 15,37 |

Beispiel A

Tabletten zu 100 mg 2-Methoxy-8-(naphth-2-yl)-purin

Zusammensetzung
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm
Dem Granulat die restlichen Hilfsstoffe zumischen und Endmischung zu Tabletten verpressen.
Tablettengewicht: 175 mg
Stempel: 8 mm

Beispiel B

Dragées zu 50 mg 2-Methoxy-8-(naphth-2-yl)-purin

Zusammensetzung:
1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

Feuchtsiebung: 1,0 mm

Trockensiebung: 1,0 mm,

Trocknung: 50°C im Umlufttrockenschrank

Granulat und restliche Hilfsstoffe mischen und zu Kernen verpressen.

Kerngewicht: 80 mg

Stempel: 6 mm

Wölbungsradius: 5 mm

Die fertigen Kerne werden auf übliche Weise mit einem Zuckerüberzug im Dragierkessel versehen.

Dragéegewicht: 120 mg

## Beispiel C

### Suppositorien zu 75 mg 2-Methoxy-8-(naphth-2-yl)-purin

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

### Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 2-Methoxy-8-(naphth-2-yl)-purin

1 Ampulle enthält:

| | |
|---|---|
| Wirksubstanz | 50,0 mg |
| Ethoxylierte Hydroxystearinsäure | 250,0 mg |
| 1,2-Propylenglykol | 1000,0 mg |
| Dest. Wasser ad | 5,0 ml |

Herstellungsverfahren:

Die Wirksubstanz wird in 1,2-Propylenglykol und ethoxylierter Hydroxystearinsäure gelöst, dann mit Wasser auf das angegebene Volumen aufgefüllt und steril filtriert.

Abfüllung: in Ampullen zu 5 ml

Sterilisation: 20 Minuten bei 120°C

Beispiel E

Tropfen zu 100 mg 2-Methoxy-8-(naphth-2-yl)-purin

| | | |
|---|---|---|
| Wirksubstanz | | 1,0 g |
| p-Oxybenzoesäuremethylester | | 0,035 g |
| p-Oxybenzoesäurepropylester | | 0,015 g |
| Anisöl | | 0,05 g |
| Menthol | | 0,06 g |
| Saccharin-Natrium | | 1,0 g |
| Glycerin | | 10,0 g |
| Ethanol | | 40,0 g |
| Dest. Wasser | ad | 100,0 ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

## Patentansprüche

1. Neue Imidazoderivate der allgemeinen Formel

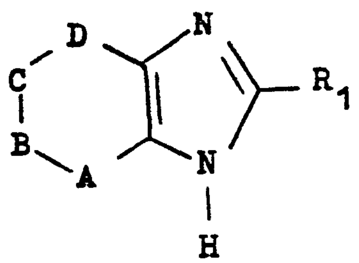

,(I)

in der

einer der Reste B oder C oder auch, wenn mindestens einer der übrigen Reste A, B, C oder D ein Stickstoffatom, eine Imino-, Hydroxymethin-, Alkoxymethin-, Halogenmethin- oder Carbonylgruppe darstellt, A ein Stickstoffatom oder eine Iminogruppe,

ein weiterer der Reste A, B, C oder D ein Stickstoffatom, eine Methin- oder Iminogruppe und

die übrigen der Reste A, B, C oder D, die gleich oder verschieden sein können, Methin-, Hydroxymethin-, Alkoxymethin-, Halogenmethin- oder Carbonylgruppen und

$R_1$ eine gegebenenfalls durch ein Halogenatom, eine Hydroxy-, Alkoxy- oder Alkylmercaptogruppe substituierete Naphthylgruppe, wobei der Alkylteil jeweils 1 bis 3 Kohlenstoffatome enthalten kann, bedeuten, deren Tautomere und deren Säureadditionssalze.

2. Neue Imidazo[4,5-d]pyridazin-4-one, Imidazo[4,5-c]pyridazin-6-one, Imidazo[4,5-c]pyridine, Imidazo[4,5-c]pyridin-4-one, Imidazo[4,5-b]pyridin-5-one, Imidazo[4,5-c]pyridin-7-one, 5-Alkoxy-imidazo[4,5-b]pyridine, Purine, Purin-2-one, 5-Hydroxy-imidazo[4,5-b]pyridine, 2-Alkoxy-purine, 2-Chlor-purine, 2-Brom-purine, Purin-6-one, Purin-2,6-dione, 6-Alkoxy-purine, 6-Chlor-purine, 6-Brom-purine, 5-Chlor-imidazo-[4,5-b]pyridine und 5-Brom-imidazo[4,5-b]pyridine der allgemeinen Formel I gemäß Anspruch 1, deren Tautomere und deren Säureadditionssalze.

3. Neue Imidazo[4,5-c]pyridine, Imidazo[4,5-b]pyridin-5-one, 5-Methoxy-imidazo[4,5-b]pyridine, Imidazo[4,5-c]pyridin-4-one, Purine, Purin-2-one, 2-Methoxy-purine, Purin-2,6-dione, 5-Chlor-imidazo[4,5-b]pyridine, 2-Chlor-purine und Imidazo-[4,5-d]pyridazin-4-one der allgemeinen Formel I gemäß Anspruch 1, wobei $R_1$ eine in 3-Stellung gegebenenfalls durch ein Chloratom, eine Hydroxy-, Methoxy-, n-Propoxy- oder Methylthiogruppe substituierte Naphth-2-yl-gruppe darstellt, deren Tautomere und deren Säureadditionssalze.

4. 2-Methoxy-8-(naphth-2-yl)-purin.

5. 2-(3-Chlor-naphth-2-yl)-1H-imidazo[4,5-b]pyridin-5-on.

6. Physiologisch verträgliche Säureadditionssalze mit anorganischen oder organischen Säuren der Verbindungen gemäß den Ansprüchen 1 bis 5.

7. Arzneimittel, enthaltend eine Verbindung gemäß den Ansprüchen 1 bis 5 oder ein physiologisch verträgliches Säureadditionssalz gemäß Anspruch 6 neben einem oder mehreren inerten Trägerstoffen und/oder Verdünnungsmitteln.

8. Verwendung einer Verbindung gemäß den Ansprüchen 1 bis 5 oder eines physiologisch verträglichen Säureadditionssalzes gemäß Anspruch 6 zur Behandlung von Herzinsuffizienzen.

9. Verfahren zur Herstellung eines Arzneimittels, dadurch gekennzeichnet, daß auf nichtchemischem Wege eine Verbindung gemäß den Ansprüchen 1 bis 5 oder ein physiologisch verträgliches Säureadditionssalz gemäß Anspruch 6 in einen oder mehreren inerten Trägerstoffen und/oder Verdünnungmitteln eingearbeitet wird.

10. Verfahren zur Herstellung der neuen Imidazoderivate der allgemeinen Formel I gemäß den Ansprüchen 1 bis 6, dadurch gekennzeichnet, daß

a) eine gegebenenfalls im Reaktionsgemisch hergestellte Verbindung der allgemeinen Formel

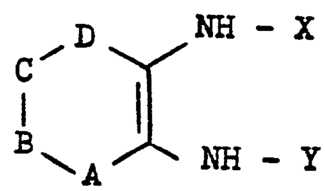

,(II)

in der
A bis D wie in den Ansprüchen 1 bis 5 definiert sind, einer der Reste X oder Y ein Wasserstoffatom und der andere der beiden Reste X und Y oder beide Reste X und Y eine Gruppe der Formel

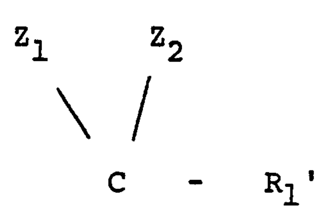

darstellen, in der

$R_1'$ die für $R_1$ in den Ansprüchen 1 bis 5 erwähnten Bedeutungen besitzt oder eine durch eine Hydroxygruppe substituierte Naphthylgruppe, in der die Hydroxygruppe durch einen Schutzrest geschützt ist,
$Z_1$ und $Z_2$, die gleich oder verschieden sein können, gegebenenfalls substituierte Aminogruppe oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung von Verbindungen der allgemeinen Formel I, in der mindestens einer der Reste A, B, C oder D eine Carbonylgruppe darstellt, ein Rest von einer Verbindung der allgemeinen Formel

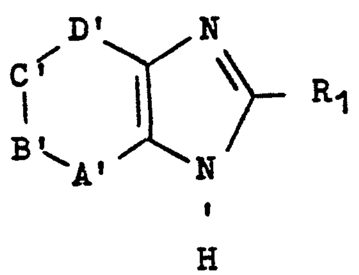
,(III)

in der
$R_1$ wie in den Ansprüchen 1 bis 5 definiert ist und mindestens einer der Reste A', B', C' oder D' eine Halogenmethingruppe oder eine durch eine Schutzgruppe geschützte Hydroxymethingruppe wie die Methoxymethin-, Äthoxymethin- - oder Benzoyloxymethingruppe und
die übrigen der Reste A', B', C' oder D' die für A, B, C oder D in den Ansprüchen 1 bis 5 erwähnten Bedeutungen besitzen, abgespalten wird und

gewünschtenfalls eine so erhaltene Verbindung der allgemeinen Formel I in ihr Säureadditionssalz, insbesondere in ihr Säureadditionssalz mit einer physiologisch verträglichen anorganischen oder organischen Säure übergeführt wird.